# EUROPEAN PATENT SPECIFICATION

(11) **EP 4 528 271 B1**
(45) Date of publication and mention of the grant of the patent: **25.02.2026**
(21) Application number: 23198981.5
(22) Date of filing: 22.09.2023
(51) Int. Cl.: G01N 33/15

(54) **DISSOLUTION TESTING APPARATUS WITH A COMMON HEATING CHANNEL**
AUFLÖSUNGSPRÜFGERÄT MIT EINEM GEMEINSAMEN HEIZKANAL
APPAREIL D'ESSAI DE DISSOLUTION AVEC UN CANAL DE CHAUFFAGE COMMUN

(43) Date of publication of application: 26.03.2025
(73) Proprietor: SOTAX AG, 4147 Aesch (CH)
(72) Inventor: Benz, Rolf, Aesch 4147 (CH); Gasser, Stefan, 4147 Aesch (CH); Saner, Ivan, 4147 Aesch (CH)
(74) Representative: Braunpat AG

(56) References cited:
- CN-A- 113 189 286
- GB-A- 2 449 150
- US-B1- 10 732 080
- US-B2- 7 495 195

## Description

### Technical Field

The invention relates to a dissolution testing apparatus used for drug release testing and dissolution testing, comprising a heating arrangement for tempering one or more testing cells, particularly one or more flow-through cells of a flow-through cell instrument or apparatus.

### Background of the invention

Dissolution testing is a compendial in vitro test for example used in pharmaceutical research and development to characterize the release rate of an Active Pharmaceutical Ingredient (API) from a formulated dosage form, under reproducible conditions, in correlation with its in vivo performance. Once an innovator product dossier has been approved by the addressed market authorities, dissolution testing is then used to assess batch-to-batch consistency of the manufactured product according to its submitted specification and according to general testing conditions described by authorities. Dissolution is also used by generic companies to establish the bioequivalence of their products, without redoing clinical studies initially done by the innovator: doing so they obtain a biowaiver.

Pharmaceutical formulations include granules, tablets, capsules, gels, creams, ointments, suspensions, suppositories, patches, microspheres, implants and other long acting injectables as micro and nano suspensions and others. "Dissolution testing" is a term used for solid dosage forms and the more general term: "Drug release testing" is used for other dosage forms, including coated medical devices. Outside of the pharmaceutical segment, dissolution testing apparatuses are also used in applications such as nicotine release from smokeless tobacco products and for persistence tests of stone wool fibers in lung fluids. The first interest of a dissolution testing apparatus in form of a flow-through cell dissolution instrument lies in its flexibility as the cell and its inside arrangement can be adapted to the tested formulation structure and challenges.

Dissolution testing modalities for solid dosage forms and apparatus involved are described in a general dissolution chapter harmonized between European, US and Japanese Pharmacopeias: the USP <711>. The flow-through cell dissolution is also described as method 6 in the Chinese Pharmacopeia.

Standardized methods use for example a paddle apparatus for stirring substances in a container as for example described in EP 0746759 A1, a basket apparatus for holding the substance in a basket as for example described in US 5816701 A, or a flow-through apparatus providing dissolution medium flowing through a cell containing the substance as for example described in WO 2013/003518 A1. The dimensions and tolerances of such apparatuses are specified precisely. Critical test parameters that have to be monitored periodically during use include for example volume and temperature of the dissolution medium, rotation speed and flow rate of medium.

The flow-through apparatus is used for a wide range of objects to be tested. The flow-through cell dissolution principle consists in flowing dissolution medium at defined temperature and flow rate onto a cell containing the dosage form to be tested. The total volume of dissolution medium is based on the solubility of the active pharmaceutical ingredient respectively substance, to be dissolved and the test duration is ideally correlated to the In vivo performance of the tested product. Therefore, the temperature of the media (from 32°C for transdermal products to 37°C for oral products and up to higher temperatures in the scope of accelerated testing) flowing into the cell has to be controlled all through the test. The cell temperature control and its efficiency are linked to two processes: the temperature control of the media flowing into the cell (at different possible flow rates as the flow rate is a method parameter) and the ambient temperature of the chamber the cell stays in.

The flow-through apparatus can be realized as an open system or a closed system as for example illustrated in EP 0049293 A1. The open system uses dissolution medium that is pumped, in all channels, from a reservoir through the cells and collected or analysed after the cells. The open system is for example recommended for poorly soluble drugs which require a high volume of media or for pH changes to be performed for example in order to mimic the Gastro-Intestinal tract. In a closed system, the media is pumped from one single reservoir per channel, through the cell and brought back to the reservoir. These individual loops for each channel allow an accumulation of dissolved active ingredient in the reservoirs which can be sampled or analysed.

In general, a dissolution testing apparatus is composed of multiple channels to allow at least the simultaneous testing of seven channels (six samples and a reference). Therefore, the seven channels have to be qualified in a documented procedure (Installation Qualification, Operational Qualification) prior to any use of the apparatus to prove that they allow an equivalent testing on all channels, as finally samples will be compared to specifications as they are representing the entire manufactured batch. The temperature in each cell (as the flow rate of media entering) has therefore to be accurate and consistent all over the test duration in order that the test only detects sample variations.

Historically, the heating systems which guarantee the right cell temperature are individual water jackets alimented in water by a temperature-controlled water bath. The same source of heat is used for the temperature regulation of entering media and cells. Often times, the cells are for example provided with heating systems comprising individual heating elements or heating units for each cell, as for example shown in WO 2010/059643 or GB 2 449 150 A. Further, an air stream or an air bath may be applied to each cell for tempering the cell and the dissolution medium, respectively, as for example shown in US 2015/0358587 A1. The cells can be associated to temperature sensing elements, which are connected to heat control systems for controlling the temperature in the cell.

Additionally, CN 113189286 A shows a dissolution apparatus wherein multiple flow through cells are arranged in a common chamber and heated simultaneously by hot air streaming into the common chamber.

However, the known dissolution testing apparatuses oftentimes are slow or complicated in adjusting the temperature of the dissolution medium in the cells. Particularly, heating arrangements using a water bath are difficult to control in a short period of time. Further, heating elements applied to each individual vessel as disclosed in the state of the art require additional application and maintenance steps for preparing and performing the testing. Most apparatuses are designed as an open system or a closed system, and they are not set up to easily switch between an open and closed configuration. Such shortcomings impact efficient quality control, compliance monitoring and product optimization.

It is an object of the invention to provide a dissolution testing apparatus for dissolving a dissoluble substance in a dissolution medium, that enables accurate and precise temperature adjustment of the dissolution medium, allows for reliable temperature control of individual dissolution cells, provides a simple and flexible set up and ensures fast and reproducible testing.

### Summary of the invention

These and other objects, which will appear from the description below, are achieved by a dissolution testing apparatus set forth in the appended independent claim. Preferred embodiments are defined in the dependent claims.

A dissolution testing apparatus for dissolving a dissoluble substance in a dissolution medium according to the present invention comprises at least one reservoir for the dissolution medium, several dissolution cells each designed for holding a dissoluble substance to be tested and a pump device for adding dissolution medium to the cells. The several cells are arranged in a common heating channel. A heater is connected to the heating channel for heating a gaseous heating medium, for example air, in the heating channel. Thus, all the cells in the heating channel will be tempered simultaneously at a temperature level established by the heater in the common heating channel. The cells are in direct interaction with the tempered ambient environment in the heating channel for fast transfer of thermal energy into the cells and to the dissoluble substance and the dissolution medium therein. It is not necessary to provide individual heating jackets for each cell.

According to the invention, a ventilator is arranged in the heating channel for creating a heated air or gas stream along the cells. The ventilator can be arranged in front of or behind the heater to blow heated air towards and along the cells in the heating channel. The heated stream created by the ventilator ensures uniform distribution of thermal energy within the common heating channel along the cells. According to the present invention, the dissolution cells are aligned along a longitudinal axis of the heating channel in the flow direction of the heated medium stream. This arrangement supports efficient interaction with the tempering stream and allows a compact design.

In one embodiment of the dissolution testing apparatus the heating channel is realized as a heating channel loop comprising a first elongated section accommodating the several cells and a second elongated section accommodating the heater and the ventilator. The several cells can be aligned along a longitudinal axis of the first elongated section of the heating channel. The heater and the ventilator can be aligned along a longitudinal axis of the second elongated section of the heating channel. The heating channel loop provides an enclosed space for the gaseous heating medium to be heated. The ventilator provides circulation of the heating medium within the heating channel. The loop design reduces the energy required to heat the heating channel to a desired temperature level and facilitates retaining the temperature level.

Advantageously, the heating channel comprises an opening along the length of the elongated section housing the dissolution cells and a cover for closing the opening for providing access to the several cells. The opening is used for inserting and removing dissolution cells

In one embodiment of the dissolution testing apparatus the heater comprises a plurality of flat heating elements, like heating plates or heating grids, arranged in the heating channel along a longitudinal axis of the heating channel. The flat surface of the heating elements runs along the length of the heating channel. This avoids disturbances of the heating stream and allows an extended time of contact between the heating medium and the heating elements, which results in efficient heating of the heating medium.

In a further embodiment of the dissolution testing apparatus according to the present invention guiding plates are located in the heating channel upstream of the several cells for supporting a laminar flow along the cells. For example, guiding plates are arranged in curved section and/or in front of the cell section of the heating channel. The guiding plates are arranged in parallel or concentrically with respect to a radius of the curved section. Preferably, a set of guiding plates is equally distanced along the width of the heating channel. For example, 3 to 8 guiding plates, depending on the size of the channel, are spread across the heating channel.

In a still further embodiment of the dissolution testing apparatus the heating channel comprises fluid passages for dissolution medium conduits in fluid connection with cell inlets for providing dissolution medium into the cells. Thus, the dissolution cells can be placed in the heating channel and dissolution medium can be added to the cells as needed.

Advantageously, a dissolution medium heating module is provided as a flow-through module in-line with a dissolution medium reservoir outside the heating channel and the dissolution cells inside the heating channel. The dissolution medium heating module pre-heats the dissolution medium before administering the medium into the dissolution cells. Thus, the dissolution medium can be heated to a desired temperature outside the dissolution cell and the heating channel. Once the heated dissolution medium is transferred into the dissolution cells in the heating channel, the heated gaseous medium stream keeps the dissolution medium at the pre-heated temperature and allows for quick adjustments of the temperature if needed.

In one embodiment of the dissolution testing apparatus according to the invention the dissolution medium heating module comprises a heating chamber, which is connect or connectable to the dissolution medium reservoir, for example through a pump device, for receiving the dissolution medium. At least one planar heating surface is arranged at the heating chamber for heating the dissolution medium therein. The heating chamber is advantageously realized as a two-dimensional heating loop structure comprising a medium inlet at one end of the heating loop structure and a medium outlet at an opposite end of the heating loop structure for receiving and dispensing dissolution medium. The at least one planar heating surface is provided adjacent to the heating loop structure for emitting and transferring thermal energy to the heating loop structure. The dissolution medium heating module allows for fast and precise heating of the dissolution medium while it continuously flows into the dissolution cells in the heating channel.

Preferably, the dissolution testing apparatus is designed for a closed loop configuration and/or an open loop configuration, like known for a USP 4 apparatus, wherein the dissolution cells are a flow-through cells comprising a cell inlet for the dissolution medium and a cell outlet for a sample solution containing dissolution medium and dissolved substance. In the closed loop configuration, the sample solution can be returned as dissolution medium into the flow-through cell to accumulate dissolved active in the reservoir. In the open loop configuration, the sample solution is extracted in fractions and then transferred into vials for analytical testing or analysed on-line.

As mentioned above, for this embodiment of the dissolution testing apparatus the heating channel comprises fluid passages for dissolution medium conduits in fluid connection to an in-flow conduit into the cell and an out-flow conduit out of the cell. For example, a lower and an upper wall of the heating channel located below and above the flow-through cells comprise connection ports for establishing a fluid communication to the in-flow conduit and the out-flow conduit. Outside the heating channel the fluid passages of the channel may connect to a dissolution medium reservoir, or the dissolution medium heating module as mentioned above, and to a sample solution conduit transferring sample solution for extraction or testing.

In the heating channel the flow-through cells are preferably arranged such, that a flow of dissolution medium through the cells is perpendicular to a heated medium stream in the heating channel. This simplifies the positioning of the heating channel in the dissolution testing apparatus and the positioning of the flow-through cells within the channel.

However, the heating channel is also suitable for heating a dissolution medium used in other cell configurations of a dissolution testing apparatus, like filled vessels instead of flow-through cells.

In a still further embodiment of the dissolution testing apparatus according to the invention at least one temperature sensor is provided in the heating channel and/or at one or more of the dissolution cells. For example, a temperature sensor can be positioned at the beginning of the elongated section housing the dissolution cells and/or at the end of that section. The temperature sensors may provide instant temperature monitoring of the temperature conditions in the heating channel and directly at the dissolution cells, which allows for precise dissolution testing.

Preferably, the dissolution testing apparatus according to the invention comprises a control unit connected to at least one temperature sensor for controlling the heater and/or the ventilator for example according to temperature measurements of the temperature sensors. Further, the control unit may control the dissolution medium heating module for adjusting an input temperature of the dissolution medium into the dissolution cells. The control unit also controls the flow rate of the dissolution medium to ensure constant and continuous medium input to the dissolution cell.

The dissolution testing apparatus according to the invention improves the reproducibility of the assessment of dissolution qualities of dissoluble substance by assuring compliance to test requirements. The dissolution testing apparatus assists in providing consistent dissolution quality for dissolving substances, particularly of drug substances. Also, it provides a predictive measure of the efficacy of API substances and other drug-based applications due to accurate temperature setting of the dissolution medium.

### Brief description of the drawings

Preferred embodiments of the invention will be described in the accompanying drawings, which may explain the principles of the invention but shall not limit the scope of the invention. The drawings illustrate:
Fig. 1: an overview of operation modules of a dissolution testing apparatus according to the present invention,
Fig. 2: an inside view of a dissolution module of the dissolution testing apparatus of figure 1 comprising dissolution medium heating modules according to the present invention,
Fig. 3: a three-dimensional view of the dissolution medium heating module according to the invention,
Fig. 4a: a longitudinal view cut through the dissolution medium heating module of figure 3,
Fig. 4b: a cut through line B-B in Figure 4a of the dissolution medium heating module of figure 3,
Fig. 5: a schematical top view of a heating arrangement for heating a cell heating channel of a dissolution testing apparatus according to the present invention,
Fig. 6a -6c: illustrative views of three operating modes of the dissolution testing apparatus: testing mode, bypass mode and cleaning mode,
Fig. 7a & 7b: a side view and a schematical interior side view of a flow-through cell as used in a dissolution testing apparatus of the present invention, and
Fig. 8a & 8b: a schematical diagram of an open loop configuration and a closed loop configuration of a dissolution testing apparatus according to the present invention.

### Detailed description of a preferred embodiment

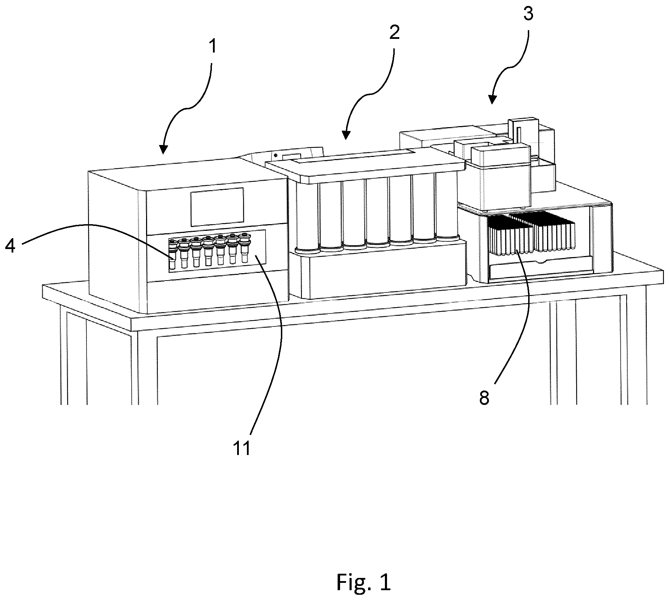

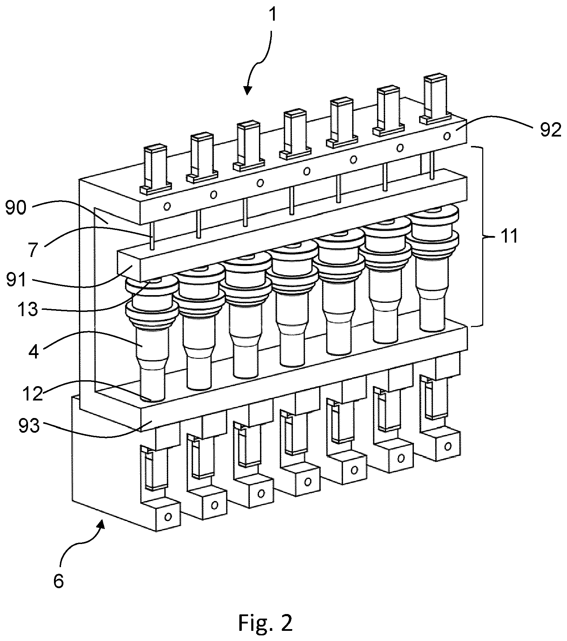

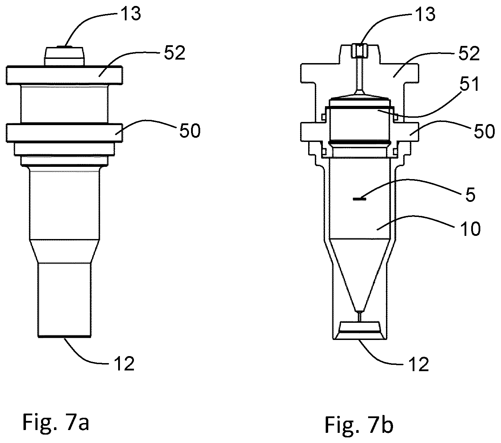

Figure 1 illustrates a setup of a dissolution testing apparatus as used in the present invention. The dissolution testing apparatus comprises a dissolution module 1, an extraction module 2 and a sample module 3. The dissolution testing apparatus can be operated in a closed mode configuration, or an open mode configuration as will be explained below. The dissolution module 1 accommodates several dissolution cells designed as flow-through cells 4, that are lined up in a cell chamber 11 of the dissolution module 1. Each cell is filled with a dissoluble substance 5 (see Figure 7a and 7b). The flow-through cells 4 are supplied with dissolution medium by dissolution medium heating modules 6 arranged below the cells in the dissolution module 1, as depicted in Figure 2. After passing the flow-through cells 4 the dissolution medium including dissolved substance exits the flow-through cells 4 as a sample solution containing dissolution medium and dissolved substance. The sample solution is transferred to the extraction module 2 via a sample solution conduit 7. In the extraction module 2 defined fractions of the sample solution are extracted from the sample solution conduit 7 into sample vials 8. Depending on the operation mode, the sample fractions in the sample vials can be tested and returned to their respective cell or the sample vials can be transferred to the sample module 3 for testing.

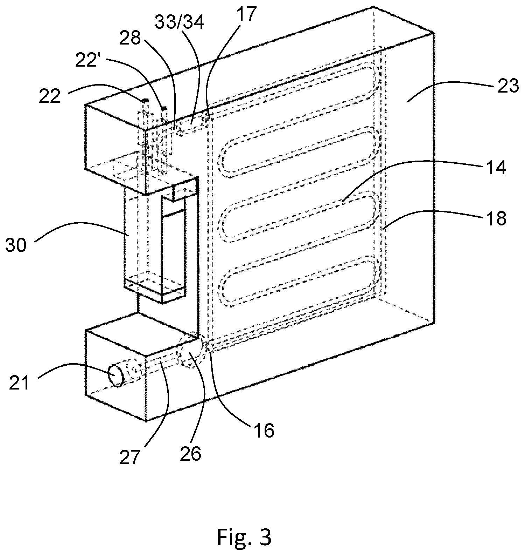

As can be seen in Figure 2, showing the interior of the dissolution module 1 of the dissolution testing apparatus according to the invention, the heating chamber 11 is realized above the dissolution medium heating modules 6 for heating the dissolution medium before the dissolution medium enters the cells 4. An assembly structure 90 in form of a mounting plate is arranged inside the dissolution module 1. On a front side, the assembly structure 90 comprises a cell rack holder 91 for holding the flow-through cells 4 upright and aligned along a longitudinal axis, a connector bar 92 comprising connector ports for connecting cell outlets 13 to the sample solution conduits 7, and a connector platform 93 for supporting the dissolution medium heating modules 6 below the platform and the flow-through cells 4 on top of the platform. The connector platform 93 comprises connector ports for connecting medium outlet lines 28 of the heating modules 6 to cell inlets 12 (see Figure 3). The connector bar 92 and the connector platform 93 extend distanced from each other and basically perpendicular from the assembly structure 90. Thus, the assembly structure 90, the connector bar 92 and the connector platform 93 create the cell chamber 11.

As shown in Figure 2 seven flow-through cells 4 are positioned in the cell chamber 11 such that a cell volume 10 (see Figure 7b) is fully exposed and accessible from all sides through walls of the flow-through cells, for example for visual inspection and interaction with conditions of the ambient environment present in the cell chamber 11. The cells comprise the cell inlet 12 at a bottom end and the cell outlet 13 at an upper end. The cell outlets 13 are in fluid communication with sample solution conduits 7, which deliver the sample solution to the extraction module 2. The bottom end of the flow-through cells 4 is positioned on the connector platform 93 for fluid connection to the dissolution medium heating modules 6 arranged below the connector platform 93 in the dissolution module 1. Each flow-through cell 4 is associated to one dissolution medium heating module 6, respectively, wherein the dissolution medium heating modules 6 are aligned next to each other below the cells.

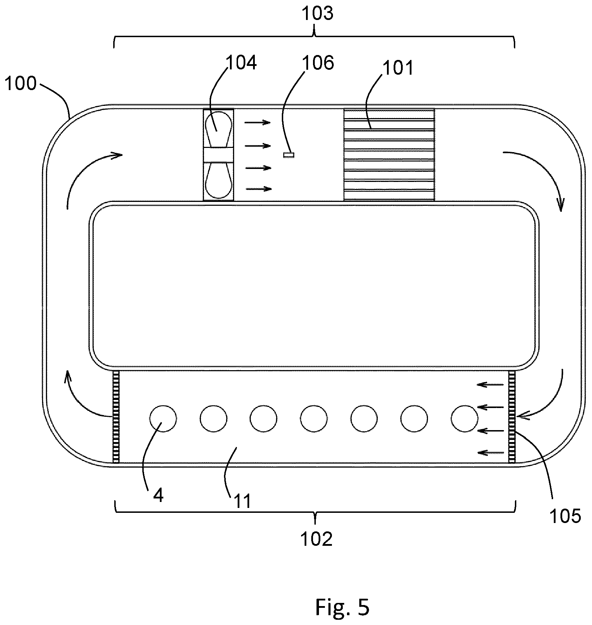

According to the present invention the several cells are arranged commonly in the heating channel 100 and a heater 101 is connected to the heating channel 100 for heating a gaseous heating medium in the heating channel, as will be described in more detail in Figure 5.

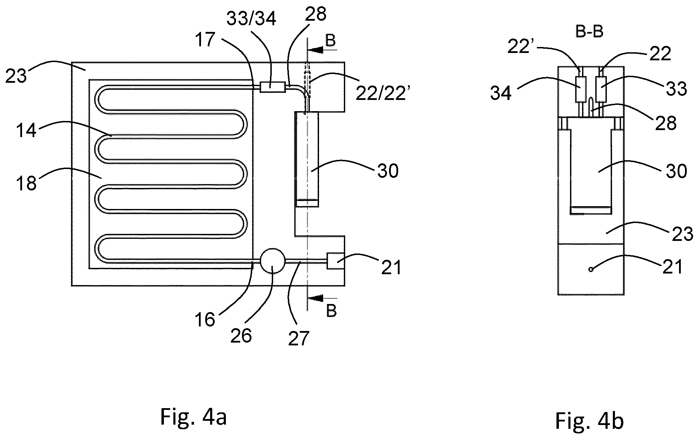

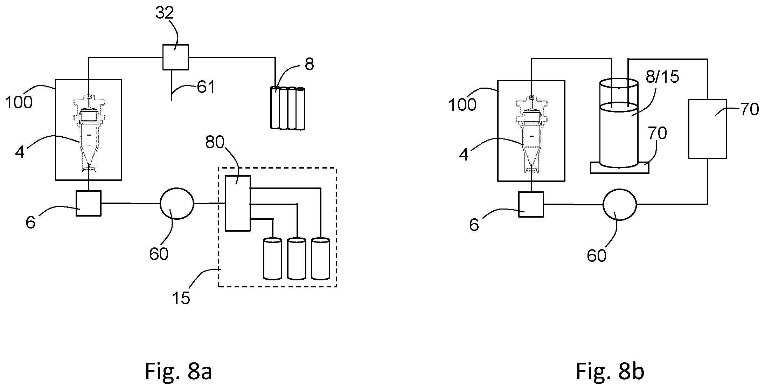

Figures 3 and 4 show the dissolution medium heating module 6 according to the present invention. The heating module comprises a heating chamber, which is realized as a two-dimensional heating loop structure 14. The heating loop structure 14 is for example designed as a medium conduit that loops in one plane for example in a serpentine pattern. The heating loop structure 14 is connected to a dissolution medium reservoir 15 (see Figures 8a and 8b) via a pump device 60 for receiving a dissolution medium. The heating loop structure 14 comprises a medium inlet 16 at one end of the heating loop structure 14 and a medium outlet 17 at an opposite end of the heating loop structure 14 for receiving and dispensing dissolution medium.

A planar heating surface in form of a heating plate 18 is arranged adjacent to the heating loop structure 14 for heating the dissolution medium in the heating loop structure 14. For example, the heating plate 18 comprises a size that covers an area of the two-dimensional heating loop structure. Alternatively, two or more heating plates or other flat heating elements or plates could be used to cover the heating loop area. The heating plate 18 is for example a ceramic plate that comprises interior heating coils. Also, silica materials or metal could be used for the planar heating surface.

The heating module receives dissolution medium from the external dissolution medium reservoir 15, which is for example located in the dissolution module 1 or in the extraction module 2 of the dissolution testing apparatus.

In the example embodiment shown in Figures 3 and 4, the dissolution medium heating module 6 comprises two housing half shells, which enclose the heating loop structure 14 and the heating plate 18 in an assembled state. One or both of the housing half shells can comprise a recess structure that corresponds to the two-dimensional heating loop structure 14 for providing, respectively receiving, the heating loop structure therein. Further, the housing half shells have an inlet through hole 21 as well as a first and second outlet through hole 22 and 22' serving as medium conduits to and from the medium inlet 16 and the medium outlet 17 of the heating loop structure 14. The two housing half shells can be screwed together to realise a heating block 23 of the heating module 6.

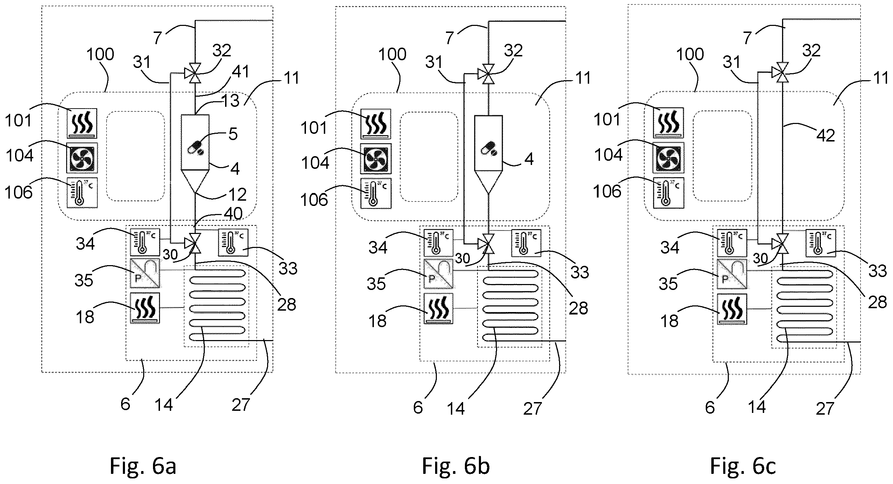

The dissolution medium heating module 6 comprises a pressure sensor 26 in a medium inlet line 27 for monitoring a pressure in the heating loop structure 14. The medium inlet line 27 is provided by the through passage of the through hole 21 and is detachably connected to the external dissolution medium reservoir 15 for example by an input port at the through hole 21. The medium outlet 17 merges into a medium outlet line 28, which enters a valve unit 30 (see Figures 6a - 6c), which can be attached to the housing. The valve unit 30 switches between parallel through passages provided by the first through hole 22 and the second through hole 22'. The first through hole 22 is connectable to the cell inlet 12 to provide tempered dissolution medium to a flow through cell 4. The second through hole 22' is connectable to a bypass conduit 31 for bypassing the cell. Each of the through holes 22 and 22' comprise a temperature sensor 33 and 34, respectively, for monitoring the temperature of the dissolution medium exiting the heating loop structure 14. The temperature sensors 33/34 and the pressure sensor 26 can be placed in the medium inlet line 27 and the through holes 22/22' when the two housing half shells are assembled to realise the heating block 23.

In an assembled state of the dissolution medium heating module 6, wherein the housing half shells are put together to form the heating block 23 the heating module comprises a slim design, wherein a thickness measured across the two half shells is only approximately half of a length or a hight of the heating module. Preferably, the thickness is only about a 1/3, more preferably only about 1/4, of the length or height. The slim design allows to provide individual dissolution medium heating modules 6 for each of the flow-through cells 4 of the dissolution testing apparatus without occupying a large space.

As will be explained in more detail for Figure 8a, a media selector 80 may be connected to the medium inlet line 27 for selectively adding a solvent to the dissolution medium before the medium enters the heating loop structure, as mentioned above. The medium can be provided to the inlet line 27 by a pump device 60.

As illustrated in Figure 2, the dissolution medium heating modules 6 are detachably mounted in the dissolution module 1 and the medium outlet line 28 of the heating loop structure 14 is detachably connected to the cell inlet 12. Thus, the heating module 6 can be replaced easily for maintenance.

Figure 5 shows a heating arrangement for heating the several dissolution cells 4 accommodated in the cell chamber 11 of the dissolution module 1. The cell chamber 11 is part of the heating channel 100 common for all cells 4. The heater 101 is arranged in the heating channel 100 for heating a gaseous medium, like air or another gas, in the heating channel 100. A ventilator 104 is arranged next to the heater 101 in the heating channel 100 for creating a heated medium stream in the heating channel and along the flow-through cells 4. In the shown example, the heating channel 100 is realized as a heating channel loop comprising a first elongated section 102 accommodating the cell chamber 11 with the several flow-through cells 4 and a second elongated section 103 accommodating the heater 101 and the ventilator 104. The first elongated section 102 and the second elongated section 103 are connected by curved sections to realize the heating channel loop. The several flow-through cells 4 are aligned along a longitudinal axis of the first elongated section 102.

The heater 101 comprises a plurality of flat heating elements, like heating plates, arranged in the heating channel 100 along a longitudinal axis of the heating channel. The air stream also flows along the longitudinal axis and can easily pass by the flat heating elements to be heated. A set of guiding plates 105 is located upstream of the several cells 4 for supporting a laminar flow along the cells in the first elongated section 102 of the heating channel 100. Further guiding plates may be located in curved section of the heating channel for supporting a laminar flow along the heater 101 and the ventilator 104.

Further, the heating channel 100 comprises fluid passages (not shown in Figure 5) for dissolution medium conduits in fluid connection with the flow-through cells 4, for example for the medium outlet line 28 of the heating module 6 or the in-flow conduit 40 that is connected to the cell inlet 12 for transmitting dissolution medium into the cell 4 and for the out-flow conduit 41 or the sample solution conduit 7 for transmitting sample solution from the cell outlet 13 to the extraction module 2.

As can be seen in Figure 2, showing the interior of the dissolution module 1 of the dissolution testing apparatus according to the invention, the heating chamber 11 of the heating channel 100 is realized above the dissolution medium heating modules 6 for heating the dissolution medium before it enters the cells 4. The assembly structure 90, the connector bar 92, the connector platform 93 and a housing side of the dissolution module 1 create the cell chamber 11 and the heating channel 100, respectively. The heater and the ventilator may be located on a back side of the assembly structure 90.

In the area of the elongated section 102 of the heating channel 100 representing the sample chamber 11, the dissolution module housing comprises the opening along the length of the sample chamber 11 and the cover (not shown) for closing the opening. The opening and the cover provide access to the cell chamber 11 for positioning the flow-through cells therein and connecting them to the connection ports. The cells are surrounded by the heated medium stream and the flow of the dissolution medium through the cells is perpendicular to the heat stream in the heating channel 100, which allows for uniform exposure to the heated air.

Initially, the dissolution medium is quickly and effectively heated to a desired temperature by the heating modules 6, while the dissolution medium is in flow to the flow-through cells 4. The heating loop structures 14 of the heating modules 6 are arranged in-line of the dissolution medium flow between the dissolution medium reservoir 15 and the flow-through cells 4, wherein the medium flow is generated by the pump device. That means the heating modules 6 are arranged in series with their associated cells 4. Then, the dissolution medium is kept at the temperature achieved by the heating modules 6 while the medium flows through the cells 4 by the heated medium stream in the heating channel 100. This set up ensures a precise continuous flow of medium through the cells with a predetermined constant temperature and enables flexible and predictive dissolution testing of dissoluble substances.

However, the dissolution testing apparatus according to the present invention does not depend on the heating modules 6. The dissolution medium can easily be heated by the heated medium stream in the heating channel 100 as provided by the present invention once the dissolution medium has entered the dissolution cells. The several cells are exposed commonly to the heated stream and the dissolution medium therein is heated uniformly for all cells.

Figures 6a to 6c describe different operations of the dissolution testing apparatus according to the present invention, schematically illustrating a flow of the dissolution medium through the dissolution medium heating module 6 and the heating channel 100.

The common heating channel comprises at least one temperature sensor 106 in the first elongated section 102 of the heating channel 100, which houses the several flow-through cells 4. Further temperature sensors can for example be located at or near at least one of the flow-through cells 4 and in the area of the heating channel accommodating the ventilator 104. A control unit at the heating channel and/or a controller of the dissolution testing apparatus may receive information data of the temperature sensors for controlling the heater 101 and/or the ventilator 104 according to a desired temperature in the heating chamber 11.

Further, the dissolution medium heating module 6 may comprise control sensors for monitoring and controlling the operation of the heating module 6 and the dissolution testing apparatus. For example, at least one temperature sensor may be provided for monitoring a temperature of the dissolution medium while it is flowing through the heating module. A temperature sensor may for example be provided at the medium inlet line 27. A temperature sensor 33 may for example be provided at the medium outlet line 28. Further, at least one pressure gauge 35 or pressure sensor may be arranged for example in the medium inlet line 27 and/or the medium outlet line 28, which could provide pressure measurements for controlling the pump 60. A control unit 29 controls the at least one heating plate 18 of the heating module 6 and the pump operation, as will be explained below.

As mentioned earlier, dissolution medium is provided by the medium inlet line 27 and enters the heating loop structure 14, wherein it is heated by the heating plate 18 while flowing through the structure. The temperature sensor 33 measures a temperature and the pressure gauge 35 measures a pressure of the dissolution medium in the medium outlet line 28. The bypass valve 30 arranged in the medium outlet line 28 dispenses the dissolution medium ether to an in-flow conduit 40 connected with the cell inlet 12 or to the bypass conduit 31. A temperature sensor 34 measures a temperature in the bypass conduit 31. In the heating channel 100, the dissolution medium is already heated by the heating module 6, flows through the flow-through cell 4 and dissolves the dissoluble substance 5 therein, which results in a sample solution containing dissolution medium and a portion of the substance dissolved therein. The sample solution exits the flow-through cell 4 through the cell outlet 13 into an out-flow conduit 41. The out-flow conduit 41 and the bypass conduit 31 end in the bypass valve 32, from where medium flow continues into the sample solution conduit 7 towards the extraction module 2 and/or sample module 3.

In Figure 6a the dissolution testing apparatus is operated in a testing mode, wherein heated dissolution medium is transferred from the dissolution medium heating module 6 into the flow-through cell 4 in the heating channel 100. The temperature in the flow-through cell 4 is kept or adjusted in the heating channel, while dissoluble substance dissolves into the dissolution medium. A sample solution is emitted into the sample solution conduit 7. The sample solution can be tested while flowing through the sample solution conduit 7, for example using optical analysis testing, or is directed to the extraction module 2 for extraction into sample vessels 8 and testing.

In Figure 6b the dissolution testing apparatus is operated in a bypass mode, wherein the split valve 30 directs dissolution medium from the medium outline line 28 and the through hole 22' of the heating module 6 into the bypass conduit 31. This may be the case for example, if the flow-through cell is clogged, or in case of any other failure of the dissolution testing apparatus. From the bypass conduit 31 the bypass valve 32 directs the dissolution medium into the sample solution conduit 7, from where the dissolution medium may be discarded or returned into the dissolution medium cycle.

In Figure 6c the dissolution testing apparatus is operated in a cleaning mode, wherein the flow-through cell has been removed from the cell chamber 11. The dissolution medium or a cleaning medium may be used for cleaning the conduits of the dissolution testing apparatus. The medium runs through the heating module 6 and the bypass valve 30 directs the medium through the bypass conduit 31 and a replacement conduit 42 replacing the in-flow conduit 40, the flow-through cell 4 and the out-flow conduit 41. The bypass valve 32 directs the medium into the sample solution conduit 7.

Figures 7a and 7b show a flow-through cell 4 as it can be used for the present invention in an assembled top view and a schematical interior view. The flow-through cell 4 comprises a cell volume 10 having a generally cylindrical shape and a predefined size. The cell volume 10 is tapered towards the bottom end, which comprises the cell inlet 12, and is open at an upper end. A dissoluble substance 5 is placed in the cell volume 10. Holding structures like a basket, a bead bed or similar may be used inside the cell volume to place the dissoluble substance distanced from the bottom if required by a used testing method or a shape of a dissoluble substance. A closing ring 50 with a central through passage and a holding frame for a filter 51 is placed on the upper opening of the cell volume 10. The filter 51 can be selected according testing requirements for the tested dissoluble substance. A cap 52 comprising the cell outlet 13 is mounted on the closing ring 50 to close the cell volume 10 and keep the filter 51 in place. Alternatively, the closing ring 50 and the cap 52 can be realized a single piece, wherein the filter 51 can be arranged in the single piece before mounting the piece on the cell volume.

The cell volume 10 is made for example of glass or plastic material that is inert with respect to the dissoluble substance and comprises transparent walls for observing the dissolution process. The cell volume 10 does not need to be placed in additional containers as known from the prior art for providing heating means at the cell. The flow-through cell 4 comprises a simple design and allows for easy preparation of dissoluble substances for testing.

Advantageously, the closing ring 50 or the cap 52 may comprise an electronic chip, a temperature sensor, a positioning sensor, a flow meter, and other sensor means. The electronic chip is designed for wireless communication with the controller of the dissolution testing apparatus and transmits information data of the temperature at the dissolution volume, the cell position, the flow through the cell, etc.. The data can be used for controlling the dissolution medium heating modules 6, the heater 101 in the heating channel 100, the pumps of the apparatus, and other functions of the dissolution testing apparatus as well as for recording the testing conditions.

Figures 8a and 8b illustrate a testing method provided by the dissolution testing apparatus of the present invention in an open loop configuration and a closed loop configuration using a flow-through cell 4.

In the open loop configuration shown in Figure 8a, fresh dissolution medium is supplied by one or more medium reservoirs 15 provided by a media selector and pumped towards the flow-through cell 4 by a medium pump 60. The dissolution medium flows through the heating module 6, where it is heated to a pre-selected temperature, and into the flow-through cell 4 in the heating channel 100. The dissolution medium flows through the flow-through cell 4 and crosses the dissoluble substance 5 to create a sample solution. The sample solution flows from the flow-through cell to the bypass valve 32, which directs the sample solution to the extraction module 2 or to a waste conduit 61. In the extraction module 2 fractions of the sample solution are extracted into sample vials 8 for testing. The total amount of dissolution medium is determined by the flow rate created by the medium pump. In the open loop configuration, the total media volume is infinite. The dissolution medium heating module 6 and the heating channel 100 according to the present invention can be used advantageously to temper the flow of fresh dissolution medium. Over time the dissoluble substance diminishes and less substance dissolves into the medium. Therefore, the measured dissolution is represented by a differential curve over time showing decreasing dissolution rates.

The closed loop configuration as shown in Figure 8b works with a fixed volume of dissolution medium supplied by a dissolution medium reservoir 15. The dissolution medium is re-circulated through the flow-through cell 4 and across the dissoluble substance several times. A testing device 70, like a UV spectrophotometer, can be arranged online for testing the sample solution while it circulates in the apparatus. Alternatively, small defined samples can be extracted for testing while the majority of sample solution is re-circulated in the closed loop system. The measured dissolution is represented by a cumulative curve over time, wherein the dissolved substance accumulates in the fixed medium volume over several cycles.

In summary, the dissolution testing apparatus according to the present invention facilitates accurate and reproducible temperature conditions for simulating in vivo conditions for dissoluble substance of all forms as listed in the beginning. The dissolution testing apparatus allows for easy change of cell types without the need of changing the heating system of the apparatus. The dissolution medium heating modules and the heating channel in the dissolution module can be used for all types of dissolution cells. The dissolution testing apparatus enables versatile and automated dissolution testing and supports precise and predictive test results.

The dissolution testing apparatus according to the present invention allows for precise and repeatable measurements of the dissolution rate and dissolution amount of a dissoluble substance of an ingredient, which is important for product safety and efficacy. The dissolution testing apparatus facilitates quality control and formulation optimization, it enables accurate prediction of in vivo drug release profiles and reliable assessments of batch-to-batch consistency. Further, it plays an important role in product compliance and market release decisions.

### List of Reference Numbers

| | | | |
|---|---|---|---|
| 1 | dissolution module | 30 | bypass valve |
| 2 | extraction module | 31 | bypass conduit |
| 3 | sample module | 32 | bypass valve |
| 4 | flow-through cell | 33 | temperature sensor |
| 5 | dissoluble substance | 34 | temperature sensor |
| 6 | dissolution medium heating module | 35 | pressure gauge |
| | | 40 | in-flow conduit |
| 7 | sample solution conduit | 41 | out-flow conduit |
| 8 | sample vial | 42 | cleaning conduit |
| 10 | cell volume | 50 | closing ring |
| 11 | cell chamber | 51 | filter |
| 12 | cell inlet | 52 | cap |
| 13 | cell outlet | 60 | medium pump |
| 14 | heating loop structure | 61 | waste conduit |
| 15 | medium reservoir | 70 | testing device |
| 16 | medium inlet | 80 | media selector |
| 17 | medium outlet | 90 | assembly structure |
| 18 | heating element | 91 | cell rack holder |
| 21 | through hole | 92 | connector bar |
| 22 | through hole | 93 | connector platform |
| 23 | heating block | | |
| 26 | pressure sensor | 100 | heating channel |
| 27 | medium inlet line | 101 | heater |
| 28 | medium outlet line | 102 | first elongated section |
| | | 103 | second elongated section |
| | | 104 | ventilator |
| | | 105 | guiding plates |
| | | 106 | temperature sensor |

## Claims

1. Dissolution testing apparatus for dissolving a dissoluble substance in a dissolution medium comprising at least one reservoir (15) for the dissolution medium, several dissolution cells (4) each designed for holding a dissoluble substance to be tested and a pump device for adding dissolution medium to the cells (4) and wherein the several cells (4) are arranged in a common heating channel (100) and aligned along a longitudinal axis of the heating channel (100), wherein a heater (101) is connected to the heating channel (100) for heating a gaseous heating medium in the heating channel (100),
the apparatus being **characterised in that** a ventilator (104) is arranged in the heating channel (100) for creating a heated medium stream along the cells (4) along the longitudinal axis of the heating channel (100).

2. Dissolution testing apparatus according to claim 1, wherein the heating channel (100) is realized as a heating channel loop comprising a first elongated section (102) accommodating the several cells (4) and a second elongated section (103) accommodating the heater (101).

3. Dissolution testing apparatus according to one of the preceding claims, wherein the heater (101) comprises a plurality of flat heating elements arranged in the heating channel (100) along a longitudinal axis of the heating channel (100).

4. Dissolution testing apparatus according to one of the preceding claims, wherein guiding plates (105) are located at least upstream of the several cells (4) for supporting a laminar flow along the cells.

5. Dissolution testing apparatus according to one of the preceding claims, wherein a control unit is connected to at least one temperature sensor (106) in the heating channel (100) and/or at least one cell (4) for controlling the heater (101) and/or the ventilator (104).

6. Dissolution testing apparatus according to one of the preceding claims, wherein the several cells (4) are aligned along a longitudinal axis of an elongated section of the heating channel (100).

7. Dissolution testing apparatus according to one of the preceding claims, wherein the cells (4) are realized as flow-through cells comprising a cell inlet (12) for dissolution medium and a cell outlet (13) for dissolution medium containing dissolved substance, wherein the flow-through cells are arranged in the heating channel (100) such, that a dissolution medium flow through the cells (4) is perpendicular to a heated medium stream in the heating channel (100).

8. Dissolution testing apparatus according to one of the preceding claims, wherein the heating channel (100) comprises fluid passages for dissolution medium conduits in fluid connection with cell inlets (12) for providing dissolution medium into the cells (4).

9. Dissolution testing apparatus according to the preceding claim, wherein a dissolution medium heating module (6) is provided as a flow-through module in-line with a dissolution medium reservoir (15) outside the heating channel (100) and the dissolution cells (4) inside the heating channel (100).

10. Dissolution testing apparatus according to one of the preceding claims, wherein an elongated section of the heating channel (100) comprises an opening along the length of the elongated section and a cover for closing the opening for providing access to the several cells (4).

11. Dissolution testing apparatus according to one of the preceding claims, wherein the heating channel (100) is integrated in a dissolution module (1) of the dissolution testing apparatus.

## Patentansprüche

1. Auflösungsprüfeinrichtung zum Auflösen einer auflösbaren Substanz in einem Auflösungsmedium, die mindestens ein Reservoir (15) für das Auflösungsmedium, mehrere Auflösungszellen (4), die jeweils zum Halten einer zu testenden auflösbaren Substanz ausgelegt sind, und eine Pumpenvorrichtung zum Hinzufügen von Auflösungsmedium zu den Zellen (4) umfasst undwobei die mehreren Zellen (4) in einem gemeinsamen Heizkanal (100) angeordnet und entlang einer Längsachse des Heizkanals (100) ausgerichtet sind, wobei eine Heizung (101) mit dem Heizkanal (100) verbunden ist, um ein gasförmiges Heizmedium in dem Heizkanal (100) zu erwärmen, wobei die Einrichtung **dadurch gekennzeichnet ist, dass** ein Ventilator (104) in dem Heizkanal (100) angeordnet ist, um einen erwärmten Medienstrom entlang der Zellen (4) entlang der Längsachse des Heizkanals (100) zu erzeugen.

2. Auflösungsprüfeinrichtung nach Anspruch 1, wobei der Heizkanal (100) als Heizkanalschleife realisiert ist, die einen ersten länglichen Abschnitt (102), der die mehreren Zellen (4) aufnimmt, und einen zweiten länglichen Abschnitt (103) umfasst, der die Heizvorrichtung (101) aufnimmt.

3. Auflösungsprüfeinrichtung nach einem der vorstehenden Ansprüche, wobei die Heizvorrichtung (101) eine Vielzahl von flachen Heizelementen umfasst, die in dem Heizkanal (100) entlang einer Längsachse des Heizkanals (100) angeordnet sind.

4. Auflösungsprüfeinrichtung nach einem der vorstehenden Ansprüche, wobei sich Führungsplatten (105) mindestens den mehreren Zellen (4) vorgelagert befinden, um eine laminare Strömung entlang der Zellen zu unterstützen.

5. Auflösungsprüfeinrichtung nach einem der vorstehenden Ansprüche, wobei eine Steuereinheit mit mindestens einem Temperaturfühler (106) in dem Heizkanal (100) und/oder mindestens einer Zelle (4) zum Steuern der Heizung (101) und/oder des Ventilators (104) verbunden ist.

6. Auflösungsprüfeinrichtung nach einem der vorstehenden Ansprüche, wobei die mehreren Zellen (4) entlang einer Längsachse eines länglichen Abschnitts des Heizkanals (100) ausgerichtet sind.

7. Auflösungsprüfeinrichtung nach einem der vorstehenden Ansprüche, wobei die Zellen (4) als Durchflusszellen realisiert sind, die einen Zelleneinlass (12) für Auflösungsmedium und einen Zellenauslass (13) für Auflösungsmedium umfassen, das aufgelöste Substanz enthält, wobei die Durchflusszellen in dem Heizkanal (100) derart angeordnet sind, dass ein Auflösungsmedienstrom durch die Zellen (4) senkrecht zu einem beheizten Medienstrom in dem Heizkanal (100) verläuft.

8. Auflösungsprüfeinrichtung nach einem der vorstehenden Ansprüche, wobei der Heizkanal (100) Fluiddurchgänge für Auflösungsmedienleitungen in Fluidverbindung mit Zelleneinlässen (12) zum Bereitstellen von Auflösungsmedium in die Zellen (4) umfasst.

9. Auflösungsprüfeinrichtung nach dem vorstehenden Anspruch, wobei ein Auflösungsmedienheizmodul (6) als Durchflussmodul in Reihe mit einem Auflösungsmedienreservoir (15) außerhalb des Heizkanals (100) und den Auflösungszellen (4) innerhalb des Heizkanals (100) bereitgestellt ist.

10. Auflösungsprüfeinrichtung nach einem der vorstehenden Ansprüche, wobei ein länglicher Abschnitt des Heizkanals (100) eine Öffnung entlang der Länge des länglichen Abschnitts und eine Abdeckung zum Verschließen der Öffnung umfasst, um Zugang zu den mehreren Zellen (4) bereitzustellen.

11. Auflösungsprüfeinrichtung nach einem der vorstehenden Ansprüche, wobei der Heizkanal (100) in einem Auflösungsmodul (1) der Auflösungsprüfeinrichtung integriert ist.

## Revendications

1. Appareil de test de dissolution destiné à dissoudre une substance dissoluble dans un milieu de dissolution comprenant au moins un réservoir (15) pour le milieu de dissolution, plusieurs cellules de dissolution (4) conçues chacune pour contenir une substance dissoluble à tester et un dispositif de pompe pour ajouter du milieu de dissolution aux cellules (4) et dans lequel les plusieurs cellules (4) sont disposées dans un canal de chauffage commun (100) et alignées le long d'un axe longitudinal du canal de chauffage (100), dans lequel un dispositif de chauffage (101) est connecté au canal de chauffage (100) pour chauffer un milieu de chauffage gazeux dans le canal de chauffage (100), l'appareil étant **caractérisé en ce qu'**un ventilateur (104) est disposé dans le canal de chauffage (100) pour créer un flux de milieu chauffé le long des cellules (4) le long de l'axe longitudinal du canal de chauffage (100).

2. Appareil de test de dissolution selon la revendication 1, dans lequel le canal de chauffage (100) est réalisé comme une boucle de canal de chauffage comprenant une première section allongée (102) accueillant plusieurs cellules (4) et une seconde section allongée (103) accueillant le dispositif de chauffage (101).

3. Appareil de test de dissolution selon l'une quelconque des revendications précédentes, dans lequel le dispositif de chauffage (101) comprend une pluralité d'éléments chauffants plats disposés dans le canal de chauffage (100) le long d'un axe longitudinal du canal de chauffage (100).

4. Appareil de test de dissolution selon l'une quelconque des revendications précédentes, dans lequel des plaques de guidage (105) sont situées au moins en amont des différentes cellules (4) pour favoriser un écoulement laminaire le long des cellules.

5. Appareil de test de dissolution selon l'une quelconque des revendications précédentes, dans lequel une unité de commande est connectée à au moins un capteur de température (106) dans le canal de chauffage (100) et/ou au moins une cellule (4) pour commander le chauffage (101) et/ou le ventilateur (104).

6. Appareil de test de dissolution selon l'une quelconque des revendications précédentes, dans lequel les différentes cellules (4) sont alignées le long d'un axe longitudinal d'une section allongée du canal de chauffage (100).

7. Appareil de test de dissolution selon l'une quelconque des revendications précédentes, dans lequel les cellules (4) sont réalisées comme des cellules à circulation comprenant une entrée de cellule (12) pour le milieu de dissolution et une sortie de cellule (13) pour le milieu de dissolution contenant une substance dissoute, dans lequel les cellules à circulation sont disposées dans le canal de chauffage (100) de telle sorte qu'un flux de milieu de dissolution à travers les cellules (4) est perpendiculaire à un flux de milieu chauffé dans le canal de chauffage (100).

8. Appareil de test de dissolution selon l'une quelconque des revendications précédentes, dans lequel le canal de chauffage (100) comprend des passages de fluide pour des conduits de milieu de dissolution en connexion fluide avec des entrées de cellules (12) pour fournir du milieu de dissolution dans les cellules (4).

9. Appareil de test de dissolution selon la revendication précédente, dans lequel un module de chauffage du milieu de dissolution (6) est fourni en tant que module d'écoulement en ligne avec un réservoir de milieu de dissolution (15) à l'extérieur du canal de chauffage (100) et les cellules de dissolution (4) à l'intérieur du canal de chauffage (100).

10. Appareil de test de dissolution selon l'une quelconque des revendications précédentes, dans lequel une section allongée du canal de chauffage (100) comprend une ouverture sur la longueur de la section allongée et un couvercle pour fermer l'ouverture afin de permettre l'accès à plusieurs cellules (4).

11. Appareil de test de dissolution selon l'une quelconque des revendications précédentes, dans lequel le canal de chauffage (100) est intégré dans un module de dissolution (1) de l'appareil de test de dissolution.
